# EUROPEAN PATENT SPECIFICATION

(11) **EP 1 424 067 B1**
(45) Date of publication and mention of the grant of the patent: **02.06.2010**
(21) Application number: 01960748.0
(22) Date of filing: 08.08.2001
(51) Int. Cl.: A61K 9/12

(54) **INJECTABLE FOAM AND NOVEL PHARMACEUTICAL APPLICATIONS THEREOF**
INJIZIERBARER SCHAUM UND NEUE PHARMAZEUTISCHE ANWENDUNGEN DAFÜR
NOUVELLES APPLICATIONS PHARMACEUTIQUES DE SUBSTANCES INJECTABLES EN MOUSSE

(43) Date of publication of application: 02.06.2004
(73) Proprietor: Garcia-Olmedo Dominguez, Maria Antonia, E-18006 Granada (ES)
(72) Inventor: Garcia-Olmedo Dominguez, Maria Antonia, E-18006 Granada (ES)
(74) Representative: Dolan, Anthony Patrick
(86) International application number: PCT/ES2001/000317
(87) International publication number: WO 2003/013475

(56) References cited:
- EP-A- 0 324 938
- EP-A- 0 656 203
- WO-A-92/05806
- US-A- 4 466 442
- MONFREUX, A.: "Traitement sclérosant des troncs saphéniens et leurs collatérales de gros calibre par la méthode MUS" PHLEBOLOGIE, vol. 50, num. 3, 1997, páginas 351-353, XP002195121

## Description

### INTRODUCTION

Parenteral administration is carried out through the skin barrier, in order to introduce medicaments in tissues or organ cavities that are not directly communicated with the exterior and even to introduce them directly into the blood stream, which acts as a distribution system.

One benefit of injectables is their rapid action (almost instantaneous in intravenous administration because the medicinal substances are immediately distributed by the blood) but it can be difficult to maintain this action locally for a given time, above all in highly vascularised areas. Furthermore, it can be laborious to achieve therapeutic concentrations of a substance in poorly vascularised areas or at sites where the traditional distribution via the bloodstream is inadequate to achieve the necessary concentrations of a medicament or to retain the medicament in the selected area for the requisite time for its action to have effect.

With the drug delivery system of injectable foam we achieve a more prolonged local action, also in richly vascularised organs and even in blood vessels. Moreover, the steerability of this pharmaceutical form makes it difficult for the action to reach undesired zones.

Furthermore, the micronisation of the medicinal substances that is produced when we place them on bubbles exponentially increases their active surface area, with the result that the same therapeutic effect is achieved with lower doses. Another benefit conferred by this pharmaceutical form is the possibility of observing ultrasonographically where the medicament is sited.

This drug delivery system may be of interest in the treatment of multiple diseases where the local action of the drugs and medicinal substances injected is of value and cannot be achieved with the pharmaceutical forms in current use.

### STATE OF THE PREVIOUS TECHNIQUE

According to patent EP,A, 0 077 752 (SCHERING AKTIENGESELLSCHAFT) 1983, liquid mixtures with physiologically compatible gas bubbles have been used as a contrast medium in ultrasound diagnosis.

There have also been attempts, according to patent WO,A, 92 05806 (SINTÉTICA S.A.) 1992, to obtain more stable suspensions of microspheres filled with gas in aqueous liquids, suitable for injection as a medium to increase the echogenicity of the blood and reinforce the ability of ultrasonography to aid medical diagnoses, as for example in the detection of vascular diseases.

Microfoam containing sclerosants has also been injected for the treatment of varicose veins and outcomes have been observed to be superior to those obtained with liquid sclerosants. (WO 95/00120 J. CABRERA GARRIDO, 1995).

An article published in Phlebology, vol. 50, num. 3, 1997, pages 351-353 discloses the obtaining of a foam containing a sclerosing agent just previous to the administration thereof, and directly in the syringe to be used for the injection. However, this document does not disclose or suggest the obtaining of an injectable foam for any drug other than the sclerosing agent.

### PREPARATION

This invention refers to the preparation of an injectable foam with any medicinal substance, adding foaming agents and gases and producing it in accordance with the conditions required.

In some diseases to be treated, the therapeutic agent can be the gas used in the formation of the foam.

The foam can be produced A) by mechanical or ultrasonic whisking of the solution, B) by depressurisation of a solution that incorporates gas dissolved under pressure, C) after the release of a gas contained in a compartment that is independent of the solution to be foamed and that is released and placed in contact with the solution at the moment of its use, D) through a chemical reaction that produces the gas, etc.

In patents US-A 4.446.442, EP-A-131 540, US-A 4.276.885, procedures are revealed to manufacture solutions of microcapsules or hollow microparticles filled with gas, i.e., microspheres in which the gas is strictly encapsulated. These procedures seek a stability of the microspheres once they are injected into the blood, which allows them to resist their destruction on their intravascular journey and thus to be detected by ultrasonography in vessels that are distant from the injection site. The high stability of these suspensions of microspheres is a necessary condition for their diagnostic efficacy.

Our invention is not attempting to achieve this, but rather to transform into foam any medicinal substance in the presence of gases and foaming agents, but without this producing a dispersion of the microbubbles, which, by continuing to be united by an immaterial boundary, form a different physicochemical entity from solutions of microparticles.

Optionally, it may be of value to improve the cohesion between the bubbles with rheologic agents.

The present invention refers more specifically to an injectable foam for administering a drug, characterised in that the foam is comprised of the drug and a gas with the proviso that the foam does not comprise a sclerosing agent.

According to particular embodiments of the invention, the drug in the injectable foam is selected from the group consisting of:
- a vasodilator
- a drug acting on the cardiovascular system
- an antimycotic
- an anti-infectious agent
- an antibiotic
- a chemoterapeutic agent
- a sulphonamide
- a cytostatic
- an anaesthetic
- an anti-inflammatory
- a prostaglandin
- a corticosteroid
- a drug with hormonal action and
- an antiviral agent.

According to particular embodiments in the injectable foam of the invention the drug has been foamed in admixture with inert foaming agents.

The invention also refers to the use of the injectable foam as has been described herein for the manufacture of an injectable drug delivery system.

### APPLICATIONS

Injectable foam is of utility, among other cases, in hepatic or renal insufficiency, or in the administration of drugs with little therapeutic margin, such as cytostatics, where we wish to achieve the maximum efficacy of the medicaments with the lowest possible dose delivered as closely as possible to the target tissue.

In localised tumours, the injection of antiinflammatories or corticosteroids in foam may reduce the gastrointestinal risks produced by these agents when they are systemically administered.

In the same way, foam is beneficial in the intravenous use of medicinal substances, for example to promote the local vasodilatation of an ischaemic foot, facilitating the continuance of the injected drug in this zone for the longest possible time.

In abscesses or localised infections, we achieve with injectable foam a more prolonged action of antibiotics or chemotherapeutic antiviral agents *in situ*, rendering them more efficacious than when they are administered traditionally.

In cases of tinea unguium, given the difficulty of achieving a satisfactory action with systemic administration, it may be of interest to inject beneath the nail foam that contains antimycotic agents.

Another application of Injectable foam may be in local anaesthesia, facilitating the diffusion or delaying the distribution of the anaesthetic and thus reducing the repetition of doses.

Moreover, when the therapeutic agent is a gas, it can be maintained in contact at the required site, formed into an injectable foam with inert substances. This would be the case of the administration of oxygen in gas gangrene produced by anaerobic germs or in severe ischaemia of the extremities.

The foam may also be of special utility when the blood cannot be the transport vehicle of a medicament and when an especially intense or selective local action is required, e.g., the *in situ* use of fibrinolytics/thrombolytics at an adequate concentration in the centre of a thrombosis of an important venous trunk.

To summarise, when it is necessary to maintain the action of an injectable medicament in a given territory, the foam form can provide an increase in its local therapeutic activity, in function of the longer time of its presence, of the reduced dilution at the site required and of the greater active surface area of the medicament.

## Claims

1. An injectable foam for administering a drug, **characterised in that** the foam is comprised of the drug and a gas with the proviso that the foam does not comprise a sclerosing agent.

2. An injectable foam in accordance with claim 1, **characterised in that** the drug is a vasodilator,

3. An injectable foam in accordance with claim 1, **characterised in that** the drug acts on the cardiovascular system.

4. An injectable foam in accordance with claim 1, **characterised in that** the drug is an antimycotic.

5. An injectable foam in accordance with claim 1, **characterised in that** the drug is an anti-infectious agent

6. An injectable foam in accordance with claim 1, **characterised in that** the drug is an antibiotic.

7. An injectable foam in accordance with claim 1, **characterised in that** the drug is a chemoterapeutic agent.

8. An injectable foam in accordance with claim 1, **characterised in that** the drug or medical substance is a sulphonamide.

9. An injectable foam in accordance with claim 1, **characterised in that** the drug is a cytostatic.

10. An injectable foam in accordance with claim 1, **characterised in that** the drug is an anaesthetic.

11. An injectable foam in accordance with claim 1, **characterised in that** the drug is an anti-inflammatory.

12. An injectable foam in accordance with claim 1, **characterised in that** the drug or medical substance is a prostaglandin.

13. An injectable foam in accordance with claim 1, **characterised in that** the drug is a corticosteroid.

14. An injectable foam in accordance with claim 1, **characterised in that** the drug has hormonal action.

15. An injectable foam in accordance with claim 1, **characterised in that** the drug is an antiviral agent

16. An injectable foam in accordance with claim 1, **characterised in that** the drug has been foamed in admixture with inert foaming agents.

17. An injectable foam in accordance with claim 1, **characterised in that** the foam is for in situ use of fibrinolytics/thrombolytics at an adequate concentration in the centre of a thrombosis in an important venous trunk

18. Use of the injectable foam as claimed in any of the preceding claims, for the manufacture of an injectable drug delivery system.

## Patentansprüche

1. Injizierbarer Schaum zum Verabreichen eines Arzneimittels, **dadurch gekennzeichnet, dass** der Schaum aus dem Arzneimittel und einem Gas besteht, mit der Maßgabe, dass der Schaum kein Sklerosierungsmittel umfasst.

2. Injizierbarer Schaum nach Anspruch 1, **dadurch gekennzeichnet, dass** das Arzneimittel ein Vasodilator ist.

3. Injizierbarer Schaum nach Anspruch 1, **dadurch gekennzeichnet, dass** das Arzneimittel auf das kardiovaskuläre System wirkt.

4. Injizierbarer Schaum nach Anspruch 1, **dadurch gekennzeichnet, dass** das Arzneimittel ein Antimykotium ist.

5. Injizierbarer Schaum nach Anspruch 1, **dadurch gekennzeichnet, dass** das Arzneimittel ein Mittel gegen Infektionen ist.

6. Injizierbarer Schaum nach Anspruch 1, **dadurch gekennzeichnet, dass** das Arzneimittel ein Antibiotikum ist.

7. Injizierbarer Schaum nach Anspruch 1, **dadurch gekennzeichnet, dass** das Arzneimittel ein Chemotherapeutikum ist.

8. Injizierbarer Schaum nach Anspruch 1, **dadurch gekennzeichnet, dass** das Arzneimittel oder die medizinische Substanz ein Sulfonamid ist.

9. Injizierbarer Schaum nach Anspruch 1, **dadurch gekennzeichnet, dass** das Arzneimittel ein Zytostatikum ist.

10. Injizierbare Schaum nach Anspruch 1, **dadurch gekennzeichnet, dass** das Arzneimittel ein Anästhetikum ist.

11. Injizierbarer Schaum nach Anspruch 1, **dadurch gekennzeichnet, dass** das Arzneimittel ein Entzündungshemmer ist.

12. Injizierbarer Schaum nach Anspruch 1, **dadurch gekennzeichnet, dass** das Arzneimittel oder medizinische Substanz ein Prostaglandin ist.

13. Injizierbarer Schaum nach Anspruch 1, **dadurch gekennzeichnet, dass** das Arzneimittel ein Kortikosteroid ist.

14. Injizierbarer Schaum nach Anspruch 1, **dadurch gekennzeichnet, dass** das Arzneimittel hormonelle Wirkung besitzt.

15. Injizierbarer Schaum nach Anspruch 1, **dadurch gekennzeichnet, dass** das Arzneimittel ein antivirales Mittel ist.

16. Injizierbarer Schaum nach Anspruch 1, **dadurch gekennzeichnet, dass** das Arzneimittel in Beimischung mit inerten Treibmitteln geschäumt ist.

17. Injizierbarer Schaum nach Anspruch 1, **dadurch gekennzeichnet, dass** der Schaum für in situ Verwendung von Fibrinolytika/Thrombolytika in einer ausreichenden Konzentration in der Mitte einer Thrombose in einem wichtigen Venenstamm ist.

18. Verwendung des injizierbaren Schaums nach einem der vorstehenden Ansprüche, zur Herstellung eines Verabreichungssystems für ein injizierbares Arzneimittel.

## Revendications

1. Mousse injectable pour administrer une substance médicamenteuse, **caractérisée en ce que** la mousse se compose de la substance médicamenteuse et d'un gaz, avec pour condition que la mousse ne comprend pas un agent sclérosant.

2. Mousse injectable selon la revendication 1, **caractérisée en ce que** la substance médicamenteuse est un vasodilatateur.

3. Mousse injectable selon la revendication 1, **caractérisée en ce que** la substance médicamenteuse agit sur le système cardio-vasculaire.

4. Mousse injectable selon la revendication 1, **caractérisée en ce que** la substance médicamenteuse est un antimycosique.

5. Mousse injectable selon la revendication 1, **caractérisée en ce que** la substance médicamenteuse est un agent anti-infectieux.

6. Mousse injectable selon la revendication 1, **caractérisée en ce que** la substance médicamenteuse est un antibiotique.

7. Mousse injectable selon la revendication 1, **caractérisée en ce que** la substance médicamenteuse est un agent chimiothérapeutique.

8. Mousse injectable selon la revendication 1, **caractérisée en ce que** la substance médicamenteuse ou médicale est un sulfonamide.

9. Mousse injectable selon la revendication 1, **caractérisée en ce que** la substance médicamenteuse est un cytostatique.

10. Mousse injectable selon la revendication 1, **caractérisée en ce que** la substance médicamenteuse est un anesthésique.

11. Mousse injectable selon la revendication 1, **caractérisée en ce que** la substance médicamenteuse est un anti-inflammatoire.

12. Mousse injectable selon la revendication 1, **caractérisée en ce que** la substance médicamenteuse ou médicale est une prostaglandine.

13. Mousse injectable selon la revendication 1, **caractérisée en ce que** la substance médicamenteuse est un corticostéroïde.

14. Mousse injectable selon la revendication 1, **caractérisée en ce que** la substance médicamenteuse a une action hormonale.

15. Mousse injectable selon la revendication 1, **caractérisée en ce que** la substance médicamenteuse est un agent antiviral.

16. Mousse injectable selon la revendication 1, **caractérisée en ce que** la substance médicamenteuse a été expansée en mélange avec des agents d'expansion inertes.

17. Mousse injectable selon la revendication 1, **caractérisée en ce que** la mousse est destinée à une utilisation *in situ* de fibrinolytiques/thrombolytiques à une concentration appropriée dans le centre d'une thrombose dans un tronc veineux important.

18. Utilisation de la mousse injectable selon l'une quelconque des revendications précédentes, pour la fabrication d'un système de délivrance de substance médicamenteuse injectable.
